# EUROPEAN PATENT APPLICATION

(11) **EP 4 454 678 A1**
(43) Date of publication of application: **30.10.2024**
(21) Application number: 21969159.9
(22) Date of filing: 28.12.2021
(51) Int. Cl.: A61M 5/142, A61M 5/145, A61M 5/172

(54) **LIQUID MEDICINE INJECTION DEVICE**

(30) Priority: 24.12.2021 KR 20210187446
(71) Applicant: Eoflow Co., Ltd., Seongnam-si, Gyeonggi-do 13605 (KR)
(72) Inventor: LEE, Do Kyoung, Hwaseong-si, Gyeonggi-do 18466 (KR); KIM, Chang Jung, Seongnam-si, Gyeonggi-do 13588 (KR); YUN, Kwang Sik, Hwaseong-si, Gyeonggi-do 18466 (KR)
(74) Representative: Studio Torta S.p.A.
(86) International application number: PCT/KR2021/019987
(87) International publication number: WO 2023/120795

(57) **Abstract**

The present disclosure provides a liquid medicine injection device including: a needle part which is provided with one or more needle bodies formed to be injected into a body of a user; a reservoir part in which liquid medicine to be delivered to the needle part is contained; a driving part which is provided with a shaft part capable of performing a reciprocating motion in a preset direction and transmits power to the reservoir part such that the liquid medicine contained in the reservoir part is delivered to the needle part; and a base part in which the reservoir part, the needle part, and the driving part are installed, wherein the base part is provided with a rib part in which a guide part is formed in a shape of a groove part, and the shaft part is capable of performing the reciprocating motion in the guide part.

## Description

### Technical Field

The present disclosure relates to a liquid medicine injection device.

### Background Art

Generally, liquid medicine injection devices, such as insulin injection devices, are used to inject liquid medicine into the body of a patient. Such liquid medicine injection devices are used by professional medical staffs, such as medical doctors and nurses. However, in most cases, liquid medicine injection devices are used by the general public, such as patients themselves or guardians thereof.

Diabetic patients, especially pediatric diabetic patients, need to inject liquid medicine, such as insulin, into the bodies thereof at regular intervals. Patch-type liquid medicine injection devices that are used while being attached to the human body for a certain period of time are under development. Such liquid medicine injection devices may be used while being attached to the body of the patient, such as the abdomen or waist of the patient, in the form of a patch for a certain period of time.

Such liquid medicine injection devices need to be controlled to precisely inject liquid medicine into the body of a user, such as a patient, and it is important to precisely inject a small amount of liquid medicine through a small liquid medicine injection device.

A conventional liquid medicine injection device includes a driving part for transmitting power to a reservoir part that discharges liquid medicine to a needle part through which liquid medicine is discharged to the outside. In the process in which the driving part transmits power while a shaft part reciprocates, a shaft of the shaft part in a movement direction is distorted and straightness is deteriorated. Accordingly, power is not properly transmitted to the reservoir part, and the precision of liquid medicine flow from the reservoir part to the needle part is deteriorated.

### Disclosure

### Technical Problem

The present disclosure provides a liquid medicine injection device in which a shaft part reciprocates inside a guide part, thereby preventing a movement central axis of the shaft part from being distorted and improving straightness.

### Technical Solution

According to an aspect of the present disclosure, there is provided a liquid medicine injection device including: a needle part which is provided with one or more needle bodies formed to be injected into a body of a user; a reservoir part in which liquid medicine to be delivered to the needle part is contained; a driving part which is provided with a shaft part capable of performing a reciprocating motion in a preset direction and transmits power to the reservoir part such that the liquid medicine contained in the reservoir part is delivered to the needle part; and a base part in which the reservoir part, the needle part, and the driving part are installed, wherein the base part is provided with a rib part in which a guide part is formed in a shape of a groove part, and the shaft part is capable of performing the reciprocating motion in the guide part.

In addition, the base part may include: a first body arranged on one side of each of the reservoir part, the needle part, and the driving part; and a second body arranged on the first body and fixing positions of the reservoir part, the needle part, and the driving part.

In addition, the rib part may be formed to have a certain thickness on the second body.

In addition, the driving part may include: a driving housing having a shaft hole formed therein; the shaft part passing through the shaft hole and extending to an outside of the driving housing; and a moving part connected to the shaft part and movable in conjunction with the reciprocating motion of the shaft part.

In addition, the driving part may further include a stopper part connected to the shaft part and arranged on either side of the moving part.

In addition, a protrusion part may be formed to extend on one side of the driving housing while sharing a longitudinal central axis with the shaft part and may be provided with the shaft hole.

In addition, a reinforcement part may be formed to have a certain thickness and protrude outward on one surface of the protrusion part where the shaft hole is formed.

In addition, the liquid medicine injection device may further include a cover part that covers the base part.

In addition, the liquid medicine injection device may further include a lever part rotatably installed in the cover part and transmitting rotational power to the needle part.

In addition, the liquid medicine injection device may further include an attachment part connected to the base part and attachable to the user.

### Advantageous Effects

In a liquid medicine device according to an embodiment of the present disclosure, since a shaft part reciprocates within a guide part formed in the shape of a groove part in a rib part provided in a base part, there is an effect that may improve straightness and improve precision in transmitting power from a driving part to a reservoir part.

In addition, since the distortion of the longitudinal central axis of the shaft part is prevented, airtightness may be maintained between the outer circumferential surface of the shaft part and the inner circumferential surface of a protrusion part formed in a driving housing.

In addition, there is an effect of preventing fluid from leaking into a space between the outer circumferential surface of the shaft part and the inner circumferential surface of the driving housing due to the distortion of the longitudinal central axis of the shaft part.

The scope of the present disclosure is not limited by such an effect.

### Description of Drawings

FIG. 1 is a perspective view illustrating a liquid medicine injection device according to an embodiment of the present disclosure.
FIG. 2 is a diagram illustrating a state in which a cover part is removed in FIG. 1.
FIGS. 3 and 4 are diagrams partially illustrating a driving part arranged on a base part, according to an embodiment of the present disclosure.
FIG. 5 is a cross-sectional view taken along line I-I of FIG. 4.
FIG. 6 is a side view partially illustrating a driving part according to an embodiment of the present disclosure.
FIG. 7 is a diagram illustrating a state in which a base part is removed in FIG. 5.
FIG. 8 is a diagram partially illustrating a second body in which a rib part is formed, according to an embodiment of the present disclosure.
FIG. 9 is a plan view partially illustrating a driving part arranged on a base part, according to an embodiment of the present disclosure.
FIG. 10 is a cross-sectional view taken along line II-II of FIG. 9.

### Mode for Disclosure

The present disclosure may allow for various modifications and embodiments, and specific embodiments will be illustrated in the drawings and described in detail in the following detailed description. Effects and features of the present disclosure, and methods of achieving them will be clarified with reference to embodiments described below in detail with reference to the drawings. However, the present disclosure is not limited to the following disclosed embodiments and may be embodied in various forms.

Hereinafter, embodiments of the present disclosure will be described in detail with reference to the accompanying drawings. When describing embodiments with reference to the accompanying drawings, the same or corresponding elements are denoted by the same reference numerals, and redundant descriptions thereof are omitted.

In the following embodiments, the singular forms are intended to include the plural forms as well unless the context clearly indicates otherwise.

In the following embodiments, the terms "comprise" or "include" specify the presence of stated features or elements, but do not preclude the presence or addition of one or more other features or elements.

When a certain embodiment may be implemented differently, a specific process order may be performed differently from the described order. For example, two consecutively described processes may be performed substantially at the same time or performed in an order opposite to the stated order.

Sizes of elements in the drawings may be exaggerated or reduced for convenience of explanation. For example, since sizes and thicknesses of elements in the drawings are arbitrarily illustrated for convenience of explanation, the following embodiments are not limited thereto.

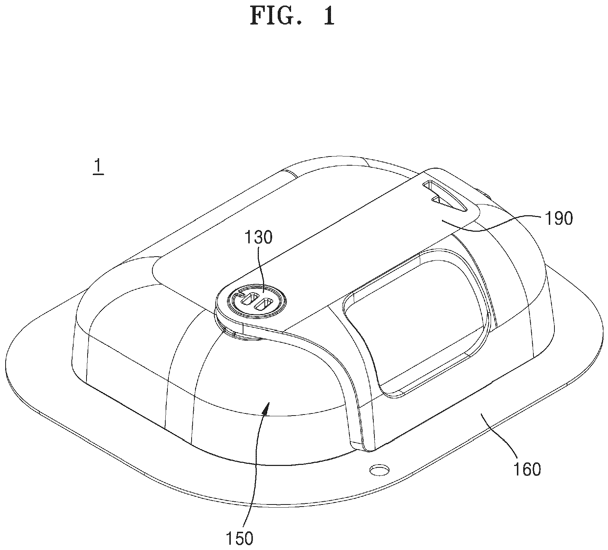

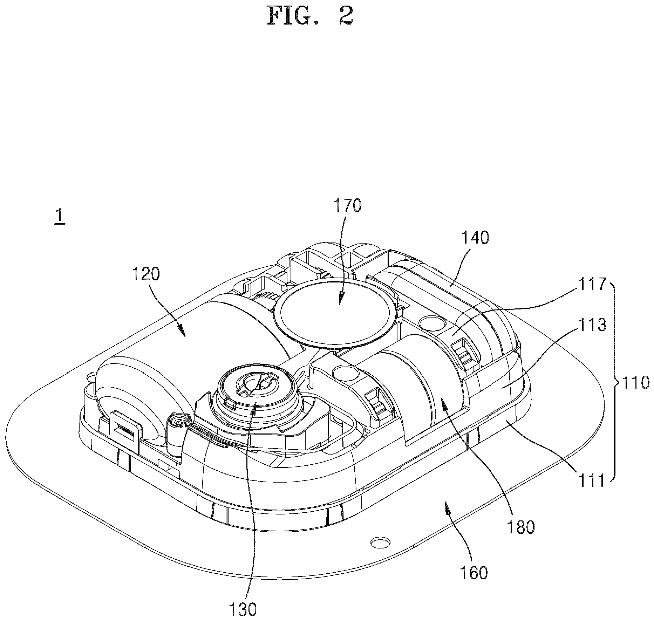

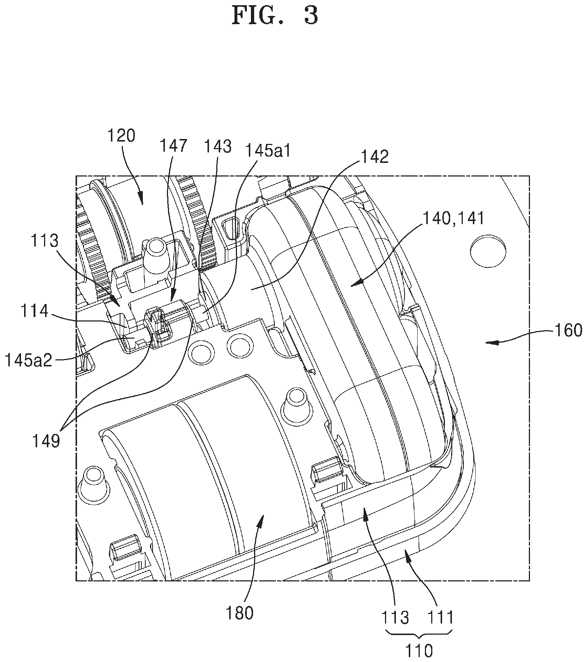

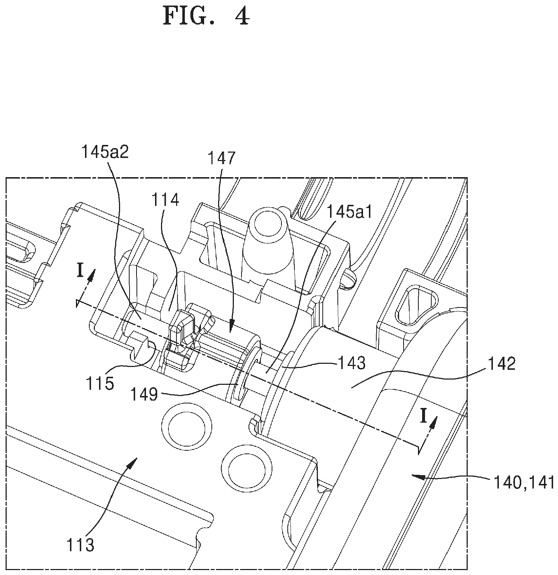

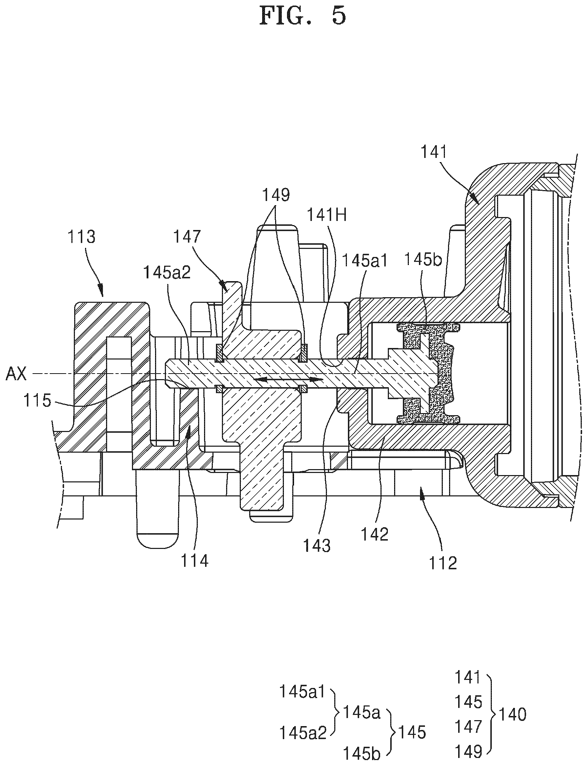

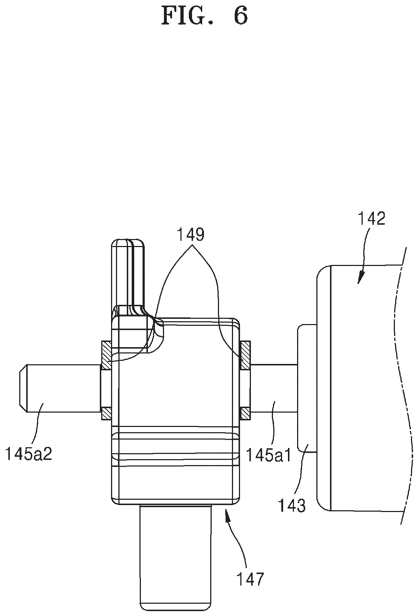

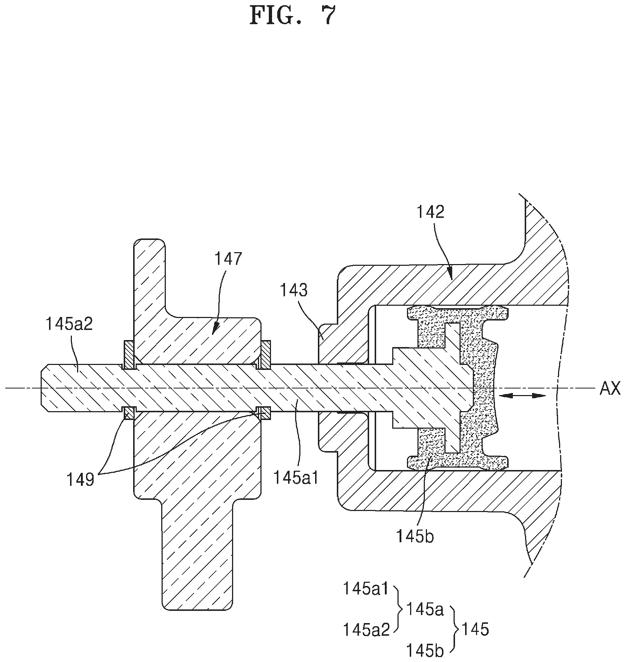

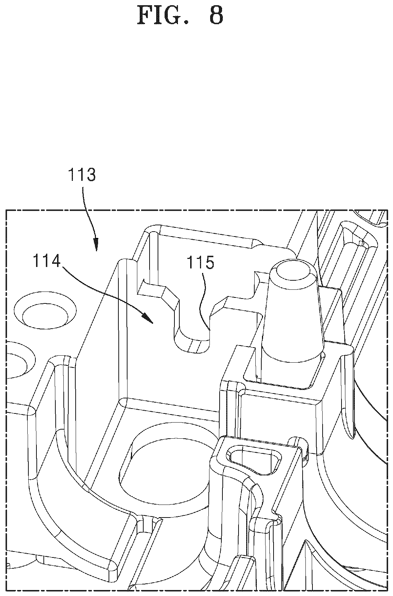

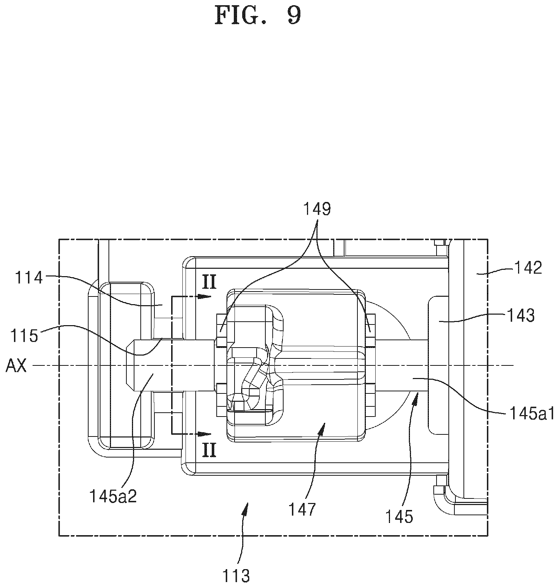

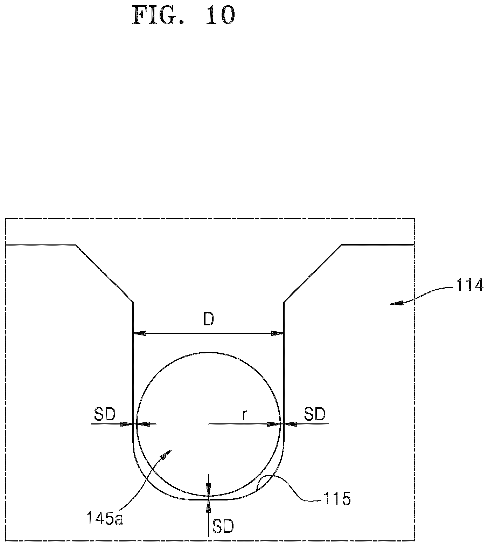

FIG. 1 is a perspective view illustrating a liquid medicine injection device according to an embodiment of the present disclosure. FIG. 2 is a diagram illustrating a state in which a cover part is removed in FIG. 1. FIGS. 3 and 4 are diagrams partially illustrating a driving part arranged on a base part, according to an embodiment of the present disclosure. FIG. 5 is a cross-sectional view taken along line I-I of FIG. 4. FIG. 6 is a side view partially illustrating the driving part according to an embodiment of the present disclosure. FIG. 7 is a diagram illustrating a state in which the base part is removed in FIG. 5. FIG. 8 is a diagram partially illustrating a second body in which a rib part is formed, according to an embodiment of the present disclosure. FIG. 9 is a plan view partially illustrating the driving part arranged on the base part, according to an embodiment of the present disclosure. FIG. 10 is a cross-sectional view taken along line II-II of FIG. 9.

Referring to FIGS. 1 to 10, a liquid medicine injection device 1 according to an embodiment of the present disclosure is attached to a subject into whom liquid medicine is to be injected and may inject liquid medicine stored therein into a user in a preset amount.

As an alternative embodiment, the liquid medicine injection device 1 may be mounted on the body of the subject into whom liquid medicine is to be injected (hereinafter referred to as a "user").

As an alternative embodiment, the liquid medicine injection device 1 may also be mounted on an animal and inject liquid medicine into the body of the animal.

The liquid medicine injection device 1 according to an embodiment of the present disclosure may be used for various purposes depending on the type of liquid medicine to be injected. For example, the liquid medicine may include insulin-based liquid medicine for diabetic patients, liquid medicine for pancreas, liquid medicine for heart, and other various types of liquid medicine.

Although not illustrated in the drawings, the liquid medicine injection device 1 may be connected to a remote device (not shown) that is connected in a wired or wireless manner.

The user may use the liquid medicine injection device 1 by operating the remote device and may monitor the usage state of the liquid medicine injection device 1. For example, the user may monitor the amount of liquid medicine injected from the liquid medicine injection device 1, the number of times liquid medicine is injected, the amount of liquid medicine stored in the reservoir, user bio information, etc. and based on this, may operate the liquid medicine injection device 1.

The remote device allows remote transmission and reception with the liquid medicine injection device 1, and the liquid medicine injection device 1 may receive a signal from an external remote device and transmit an electrical signal to a driving part 140, which will be described later.

The driving part 140 is driven to transmit power to a reservoir part 120, and liquid medicine contained in the reservoir part 120 is delivered to a needle part 130. There is an effect of allowing liquid medicine to be injected into the body of the user through a discharge path formed in the needle part 130.

In the present specification, the term "remote device" refers to a communication terminal that is capable of using an application in a wired or wireless communication environment. Here, the remote device may be a user's portable terminal.

To explain this in more detail, the remote device (not shown) may include computers (e.g., desktops, laptops, tablets, etc.), media computing platforms (e.g., cable, satellite set-top boxes, digital video recorders, etc.), handheld computing devices (e.g., personal digital assistants (PDAs), email clients, etc.), any form of mobile phone, any form of wearable device that is usable by being attached to or mounted on the body of the user, or any form of other computing or communication platforms, but the present disclosure is not limited thereto.

The liquid medicine injection device 1 and the remote device may communicate with each other through a communication network. In this case, the communication network refers to a communication network that provides a connection path that allows the remote device to transmit and receive data after connecting to a service server (not shown). Examples of the communication network may include wired networks, such as local area networks (LANs), wide area networks (WANs), metropolitan area networks (MANs), and integrated service digital networks (ISDNs), or wireless networks, such as wireless LANs, code division multiple access (CDMA), Bluetooth, and satellite communications, but the scope of the present disclosure is not limited thereto.

As an alternative embodiment, the remote device is illustrated as a single device, but the present disclosure is not limited thereto, and the remote device may include a plurality of devices capable of communicating with the liquid medicine injection device 1.

Referring to FIGS. 1 and 2, the liquid medicine injection device 1 according to an embodiment of the present disclosure may include a base part 110, a reservoir part 120, a needle part 130, a driving part 140, a cover part 150, an attachment part 160, a battery part 180, and a lever part 190.

Referring to FIGS. 1 and 2, in the liquid medicine injection device 1 according to an embodiment of the present disclosure, the cover part 150 that forms an exterior and has a hollow interior may cover the base part 110. Internal components, such as the reservoir part 120, the needle part 130, the driving part 140, and the battery part 180, which will be described later, may be arranged on the base part 110 according to an embodiment of the present disclosure.

Referring to FIGS. 2, 3, 4, 5, 8, 9, and 10, the reservoir part 120, the needle part 130, and the driving part 140, which will be described later, are installed in the base part 110 according to an embodiment of the present disclosure. The base part 110 according to an embodiment of the present disclosure may include a first body 111, a second body 113, and a third body 117.

The first body 111 according to an embodiment of the present disclosure may be in contact with the attachment part 160, which will be described later, and may be connected to the cover part 150. Specifically, the cover part 150 may cover the first body 111 and may be connected to the first body 111.

Referring to FIGS. 2 and 5, a substrate part 112 may be arranged on the first body 111 according to an embodiment of the present disclosure, and electrical signals may be transmitted to the driving part 140, the battery part 180, an alarm unit 170, etc. through the substrate part 112.

Conducting wires may be installed in the substrate part 112 according to an embodiment of the present disclosure, and the substrate part 112 may be formed as, for example, a printed circuit board (PCB).

The first body 111 according to an embodiment of the present disclosure faces the cover part 150 and is connected to the attachment part 160. The first body 111 may be arranged below the needle part 130, the driving part 140, and the battery part 180.

Referring to FIGS. 3 to 5, 8, 9, and 10, the second body 113 according to an embodiment of the present disclosure is arranged on the first body 111 and may fix the positions of internal components, such as the reservoir part 120, the needle part 130, the driving part 140, and the battery part 180.

The second body 113 covers the lower sides of the needle part 130, the driving part 140, and the battery part 180, and at least one hole part or groove part may be formed in the second body 113. There is an effect in which internal components, such as the needle part 130, the driving part 140, and the battery part 180 may be arranged in the hole part or the groove part.

Specifically, the second body 113 may cover the first body 111 on which the substrate part 112 is arranged and may provide a space in which the reservoir part 120, the needle part 130, the driving part 140, and the battery part 180 may be arranged.

This provides an effect of preventing internal components, such as the reservoir part 120, the needle part 130, the driving part 140, and the battery part 180, which are arranged between the base part 110 and the cover part 150, which will be described later, from being shaken or changed in position due to the movement of the user while the liquid medicine injection device 1 is located on the body of the user.

Referring to FIGS. 3 to 5, 8, and 9, the second body 113 according to an embodiment of the present disclosure may include a rib part 114. One end portion of the driving part 140, which will be described later, specifically one end portion of a shaft part 145 may be arranged to pass through the rib part 114.

Referring to FIGS. 3 to 5, the rib part 114 according to an embodiment of the present disclosure may be formed in the shape of a plate with a certain thickness. On the base part 110, specifically on the first body 111, the rib part 114 may divide an area where the driving part 140 is arranged into a plurality of areas.

Referring to FIGS. 3, 4, 5, 9, and 10, the rib part 114 formed in the second body 113 according to an embodiment of the present disclosure may be arranged outside the shaft part 145 provided in the driving part 140, which will be described later, specifically outside a shaft body 145a.

Referring to FIG. 8, a guide part 115 is formed in the rib part 114 in the shape of a groove part, and the shaft body 145a is arranged inside the guide part 115 and may reciprocate along a longitudinal central axis AX of the shaft part 145 in a preset direction (a left and right direction in FIG. 9).

Referring to FIGS. 3 to 5, 9, and 10, the guide part 115 formed in the rib part 114 according to an embodiment of the present disclosure may be open on one side (an upper side in FIG. 10) and may be formed in the shape of a groove part.

Since one side (the upper side in FIG. 10) of the guide part 115 is open, there is an effect in which the driving part 140 may be inserted and arranged in an area formed on the base part 110, specifically the second body 113, and the shaft body 145a may pass through the open one side (the upper side in FIG. 10) of the guide part 115 and may be arranged inside the guide part 115.

Referring to FIG. 10, a width D of the guide part 115 according to an embodiment of the present disclosure may be formed to be relatively greater than a diameter 2r of the shaft part 145 arranged inside the guide part 115, specifically the shaft body 145a.

As an alternative embodiment, the diameter 2r of the shaft body 145a may be formed to be 1 mm, and the width D of the guide part 115 may be formed to be 1.05 mm.

This provides an effect in which the inner circumferential surface of the guide part 115 formed in the rib part 114 and the shaft body 145a maintain a state of being spaced apart from each other, without coming into contact with each other.

In addition, when the driving part 140 is arranged on the base part 110, specifically the second body 113, the shaft part 145, specifically the shaft body 145a, may be easily inserted into the guide part 115 formed in the rib part 114.

In addition, since the inner circumferential surface of the guide part 115 and the outer circumferential surface of the shaft body 145a maintain a state of being spaced apart from each other, there is an effect in which it is possible to prevent friction from occurring due to the contact between the shaft part 145 and the rib part 114 and it is possible to prevent the power transmission performance of the driving part 140 to the reservoir part 120 from being deteriorated due to such friction.

Referring to FIGS. 3, 4, 9, and 10, since the shaft body 145a according to an embodiment of the present disclosure passes through the guide part 115 formed in the rib part 114 and is arranged inside the guide part 115, there is an effect of minimizing deformation of the longitudinal central axis AX, which may occur in the process in which the shaft part 145 is reciprocating along the longitudinal central axis AX by using the power generated by the driving part 140.

Referring to FIG. 9, when the rib part 114 is not formed in the second body 113, an empty space is formed between the outside of the driving part 140, specifically the shaft body 145a, and the inner circumferential surface of the inner area of the second body 113 where the driving part 140 is arranged. Due to the empty space, the movement path may deviate from the longitudinal central axis AX in the process in which the shaft body 145a is reciprocating.

Specifically, the shaft body 145a may rotate in a clockwise or counterclockwise direction with respect to a preset point on the longitudinal central axis AX on the shaft body 145a, and the angle of the longitudinal central axis AX may be changed. Since the guide part 115 that is formed in the rib part 114 in the shape of a groove part is arranged outside the shaft body 145a, there is an effect of minimizing the change in the angle of the longitudinal central axis AX and ensuring straightness of the reciprocating motion of the shaft part 145.

In addition, the inner circumferential surface of the guide part 115 formed in the rib part 114 and the outer circumferential surface of the shaft body 145a may be spaced apart from each other by a certain distance, specifically a separation distance SD. By maintaining a non-contact state between the inner circumferential surface of the guide part 115 and the outer circumferential surface of the shaft body 145a, friction caused by the contact therebetween may be minimized.

In addition, the outer circumferential surface of the shaft body 145a and the inner circumferential surface of the guide part 115 formed in the rib part 114 maintain a non-contact state while being spaced apart from each other by the separation distance SD, the driving part 140 may generate preset power and transmit the power to the reservoir part 120. Accordingly, there is an effect of allowing a fixed amount of liquid medicine to be delivered from the reservoir part 120 to the needle part 130.

Referring to FIG. 10, the separation distance SD formed between the outer circumferential surface of the shaft body 145a and the inner circumferential surface of the opposing guide part 115 according to an embodiment of the present disclosure may be formed in plurality. Points at which the separation distances SD are formed may be arranged equiangularly with respect to the center of the shaft body 145a.

Specifically, three points that are spaced apart from each other by the separation distance SD may be formed between the inner circumferential surface of the guide part 115 formed in the rib part 114 and the outer circumferential surface of the shaft body 145a. The three points may be arranged at 90-degree intervals with respect to the center of the shaft body 145a.

That is, points that are spaced apart from each other by the separation distance SD may be formed between the outer circumferential surface of the shaft body 145a and the inner circumferential surface of the guide part 115 on the left, right, and lower sides (in FIG. 10) of the shaft body 145a excluding one side (in FIG. 10) of the guide part 115 in which the opening (not denoted by reference numeral) is formed. As an alternative embodiment, the separation distance SD may be set to 0.025 mm.

Due to this, there is an effect of improving straightness by preventing the distortion of the longitudinal central axis AX and the change of the movement path when the shaft body 145a reciprocates along the longitudinal central axis AX.

In addition, since the outer circumferential surface of the shaft body 145a and the inner circumferential surface of the guide part 115 formed in the rib part 114 are arranged in a state of being spaced apart from each other, there is an effect of preventing a driving force from being reduced due to friction caused by the contact between the shaft body 145a and the guide part 115.

Referring to FIG. 9, the rib part 114 according to an embodiment of the present disclosure may be arranged outside the shaft body 145a, specifically a second part 145a2 that is the other end area opposite to a first part 145a1 that is one end area to which a shaft head 145b is connected.

This provides an effect in which the first part 145a1 of the shaft body 145a may prevent the movement central axis, that is, the longitudinal central axis AX, from being distorted by a reinforcement part 143 formed in a driving housing 141, which will be described later, and the second part 145a2 of the shaft body 145a is located inside the guide part 115 formed in the rib part 114 and may prevent the longitudinal central axis AX from being distorted.

Referring to FIGS. 2 and 3, the third body 117 according to an embodiment of the present disclosure may be arranged on the second body 113, may partially cover the upper side of the second body 113 (in FIG. 2), and may fix the positions of the reservoir part 120, the battery part 180, the needle part 130, and the driving part 140.

Referring to FIGS. 2 and 3, the reservoir part 120 according to an embodiment of the present disclosure stores liquid medicine to be injected and may receive power from the driving part 140 and deliver a fixed amount of liquid medicine to the needle part 130.

A plunger (not shown) may be arranged inside the reservoir part 120. While moving in a preset direction, the plunger may deliver liquid medicine contained in the reservoir part 120 to the needle part 130.

The reservoir part 120 may be connected to the driving part 140, and the plunger may be moved with power received from the driving part 140. Due to the movement of the plunger, a fixed amount of liquid medicine may be delivered to the needle part 130.

The reservoir part 120 and the driving part 140 may be geared to each other, but the present disclosure is not limited thereto. Various modifications are possible within the technical concept in which the power generated by the driving part 140 may be transmitted to the reservoir part 120 and liquid medicine contained in the reservoir part 120 may flow into the needle part 130.

As an alternative embodiment, the reservoir part 120 may be variously modified, such as being installed outside the base part 110, within the technical concept of delivering liquid medicine to the needle part 130.

Referring to FIG. 2, the needle part 130 according to an embodiment of the present disclosure includes one or more needle bodies (not shown) formed to be injected into the body of the user, and may be arranged on the base part 110, specifically the second body 113.

The needle body provided in the needle part 130 may pass through the base part 110 and the attachment part 160 and be inserted into the skin of the user, and may form a hollow therein such that discharged liquid medicine is injected into the user.

Referring to FIGS. 2 and 3, the battery part 180 according to an embodiment of the present disclosure may activate each component by supplying electricity to the liquid medicine injection device 1. Although the single battery part 180 is illustrated in the drawings, the present disclosure is not limited thereto, and the battery part 180 may be variously set depending on the capacity, usage range, usage time, etc. of the liquid medicine injection device 1.

The battery part 180 according to an embodiment of the present disclosure may be arranged adjacent to the driving part 140 and may supply electricity to the driving part 140. In addition, the battery part 180 may be connected to the substrate part 112 and may supply power to the substrate part 112.

Although not illustrated in the drawings, the control part may measure, based on an electrical signal measured by a sensor part (not shown), data about the driving performance of the driving part 140, the amount of liquid medicine stored in the reservoir part 120, and the amount of liquid medicine injected into the user.

Referring to FIGS. 2 to 6, 7, 8, and 9, the driving part 140 according to an embodiment of the present disclosure is provided with the shaft part 145 capable of reciprocating in a preset direction and may transmit power to the reservoir part 120 such that liquid medicine stored in the reservoir part 120 is delivered to the needle part 130.

The driving part 140 according to an embodiment of the present disclosure may include a driving housing 141, a shaft part 145, a moving part 147, and a stopper part 149.

Referring to FIGS. 3 to 5, the driving housing 141 according to an embodiment of the present disclosure has a shaft hole 141H formed on one side and has a hollow formed therein. The driving housing 141 may be arranged on the base part 110, specifically the second body 113.

Referring to FIGS. 3 to 5, a protrusion part 142 may be formed to protrude in an outward direction on the driving housing 141 according to an embodiment of the present disclosure, and the shaft hole 141H may be formed in the protrusion part 142.

Referring to FIGS. 5 and 7, a certain portion of the shaft part 145 may be arranged to pass through the shaft hole 141H in the protrusion part 142 formed to protrude from the driving housing 141 according to an embodiment of the present disclosure. The shaft part 145 may reciprocate along the longitudinal central axis AX and may repeatedly move forward and backward. Due to the reciprocating motion of the shaft part 145, there is an effect in which power is transmitted to the reservoir part 120 such that a fixed amount of liquid medicine is delivered from the reservoir part 120 to the needle part 130.

The driving part 140 according to an embodiment of the present disclosure may be electrically connected to the substrate part 112, may generate power by receiving an electrical signal from the substrate part, and may move the shaft part 145 by using the power.

The driving part 140 according to an embodiment of the present disclosure may be any type of device having a liquid medicine suction power and a liquid medicine liquid discharge power by electricity. For example, any type of pump, including a mechanical displacement micropump and an electromagnetic kinetic micropump, may be used.

The mechanical displacement micropump is a pump that uses the movement of a solid or a fluid, such as a gear or a diaphragm, to cause a pressure difference so as to induce the flow of fluid. The mechanical displacement micropump may include a diaphragm displacement pump, a fluid displacement pump, a rotary pump, and the like. The electromagnetic kinetic micropump is a pump that directly uses electrical or magnetic energy to move a fluid. The electromagnetic kinetic micropump may include an electro hydrodynamic pump (EHD), an electro osmotic pump, a magneto hydrodynamic pump, an electro wetting pump, and the like.

The driving part 140 according to an embodiment of the present disclosure may contain a fluid in the driving housing 141, and a membrane (not shown) may be arranged inside the driving housing 141.

An inner space of the driving housing 141 may be divided into a first space arranged in a direction away from the shaft hole 141H and a second space adjacent to the shaft hole 141H, and the membrane may be arranged between the first space and the second space.

Although not illustrated in the drawings, the driving part 140 may have a first electrode body and a second electrode body respectively arranged on both sides of the membrane. The first electrode body and the second electrode body may be in contact with the substrate part 112 and may receive an electrical signal from the substrate part 112.

When the driving part 140 according to an embodiment of the present disclosure receives an electrical signal from the substrate part 112, an electrochemical reaction may allow the fluid to pass through the membrane. By alternately applying different signals, the fluid contained in the driving housing 141 may flow from the first space to the second space and from the second space to the first space.

The reciprocating motion of the shaft part 145 according to an embodiment of the present disclosure may depend on the flow of fluid moving between the first space and the second space. Due to the pressure of fluid, there is an effect in which the shaft part 145 reciprocates along the longitudinal central axis.

Referring to FIGS. 3 to 7, 8, and 9, the shaft part 145 according to an embodiment of the present disclosure passes through the shaft hole 141H and extends to the outside of the driving housing 141. The shaft part 145 may include a shaft body 145a and a shaft head 145b.

The shaft body 145a is formed to extend along the longitudinal central axis AX. The shaft body 145a may include a first part 145a1 arranged inside the protrusion part 142 that passes through the shaft hole 141H and protrudes from one side of the driving housing 141, and a second part 145a2 that faces the first part 145a1 and is arranged at the outside.

The shaft head 145b may be connected to the first part 145a1 of the shaft body 145a located inside the protrusion part 142. The outer circumferential surface of the shaft head 145b may be in contact with the driving housing 141, specifically the inner circumferential surface of the protrusion part 142.

This provides an effect in which it is possible to prevent the fluid contained in the driving housing 141 from leaking between the outer circumferential surface of the shaft head 145b and the inner circumferential surface of the protrusion part 142, and the shaft body 145a may reciprocate while maintaining the longitudinal central axis AX.

The shaft head 145b according to an embodiment of the present disclosure may be formed of an elastically deformable material, and specifically, may be formed of a rubber material. There is an effect of being in close contact with the inner circumferential surface of the protrusion part 142 formed in the driving housing 141 and preventing fluid from leaking between the inner circumferential surface of the protrusion part 142 and the outer circumferential surface of the shaft head 145b.

Referring to FIGS. 4 to 7 and 9, the reinforcement part 143 with a certain thickness may be formed to protrude outward from one surface of the protrusion part 142 formed to protrude from the driving housing 141 according to an embodiment of the present disclosure.

Since the reinforcement part 143 shares a central axis with the longitudinal central axis AX and is formed to protrude with a certain thickness on the outer circumferential surface of the protrusion part 142, a section overlapping the shaft body 145a may be relatively increased.

There is an effect of surrounding the outer circumferential surface of the shaft body 145a by the thicknesses of the protrusion part 142 located outside the shaft body 145a and the reinforcement part 143 formed to protrude from the protrusion part 142 and stably guiding the reciprocating motion path of the shaft part 145, specifically the shaft body 145a.

Referring to FIG. 7, the reinforcement part 143 may be formed to protrude with a certain thickness on the outer surface of the protrusion part 142 formed in the driving housing 141 according to an embodiment of the present disclosure, and the thickness of the protrusion part 142 in the area where the reinforcement part 143 is formed to protrude may be formed to be equal to or relatively greater than the diameter of the shaft body 145a.

As an alternative embodiment, the thickness of the protrusion part 142 in the area where the reinforcement part 143 is formed to protrude, that is, the thickness of the area surrounding the shaft body 145a may be formed to be 1.25 mm.

Due to this, it may be possible to prevent the shaft body 145a from rotating at a certain angle in a clockwise direction (in FIG. 7) or a counterclockwise direction (in FIG. 7) with a preset point on the longitudinal central axis AX of the shaft body 145a as the center of rotation.

In addition, there is an effect of improving straightness by preventing the distortion of the angle of the longitudinal central axis AX, around which the shaft body 145a rotates and which is the reciprocating motion path.

In other words, the shaft part 145, specifically the first part 145a1 of the shaft body 145a, is surrounded by the reinforcement part 143 formed to protrude outward from the protrusion part 142 where the shaft hole 141H is formed, and may prevent the distortion of the longitudinal central axis AX of the shaft body 145a. The second part 145a2 of the shaft body 145a is arranged inside the guide part 115 formed in the shape of a groove part on the rib part provided in the base part 110, specifically the second body 113, and prevents the distortion of the longitudinal central axis AX of the shaft body 145a. Accordingly, there is an effect of improving straightness of the shaft part 145 when the driving part 140 generates power.

In addition, there is an effect in which power is transmitted to the reservoir part 120 as the shaft part 145 reciprocates along the longitudinal central axis AX with straightness without distortion of the reciprocating motion path, a fixed amount of liquid medicine may be delivered to the needle part 130, and liquid medicine may be delivered to the body of the user through the needle body provided in the needle part 130.

That is, since the reciprocating motion of the driving part 140, specifically the shaft part 145, ensures straightness, there is an effect in which power may be transmitted to the reservoir part 120 with a preset force and a fixed amount of liquid medicine may be injected into the body of the user from the reservoir part 120 through the needle part 130.

Referring to FIGS. 3 to 7 and 9, the moving part 147 according to an embodiment of the present disclosure is connected to the shaft part 145 and is movable in conjunction with the reciprocating motion of the shaft part 145. The moving part 147 transmits power to the reservoir part 120 and may be directly or indirectly connected to the reservoir part 120 to transmit power generated by the driving part 140 to the reservoir part 120.

As the shaft part 145 reciprocates, the moving part 147 may also move linearly, and the contact with a liquid medicine discharge part (not shown) provided in the reservoir part 120 is possible. The liquid medicine discharge part may receive power from the driving part 140, specifically the moving part 147, and may move the plunger such that liquid medicine contained in the reservoir part 120 is delivered to the needle part 130.

In FIGS. 3 and 4, the stopper part 149 according to an embodiment of the present disclosure is connected to the shaft part 145, specifically the shaft body 145a, and may be arranged on either side of the moving part 147.

A groove part (not denoted by reference numeral) may be formed along the circumference of the shaft body 145a such that the stopper part 149 may be fixed in position. The groove has a certain depth, and the stopper part 149 is seated on the groove part. Accordingly, there is an effect of preventing the moving part 147 from moving on the shaft body 145a along the longitudinal central axis AX of the shaft body 145a.

Referring to FIGS. 1 and 2, the cover part 150 according to an embodiment of the present disclosure may be arranged on the upper side of the base part 110 (in FIG. 2), and the attachment part 160 may be arranged on the other side (the lower side in FIG. 2) opposite thereto.

Referring to FIGS. 1 to 3, the attachment part 160 according to an embodiment of the present disclosure is connected to the base part 110 and may be formed to be attachable to the user.

Specifically, the attachment part 160 is connected to the base part 110, specifically one side of the first body 111 (the lower side in FIG. 2) and an adhesive material may be applied to the attachment part 160. Due to this, there is an effect in which the attachment part 160 may be attached to the skin of the user, and the position of the liquid medicine injection device 1 coming into contact with the body of the user may be fixed.

As an alternative embodiment, an adhesive material may be applied to both sides (the upper and lower sides in FIG. 2) of the attachment part 160. One side (the upper side in FIG. 2) of the attachment part 160 may be in contact with and connected to the first body 111, and the other side (the lower side in FIG. 2) of the attachment part 160 opposite thereto may be in contact with and attached to the body of the user.

As an alternative embodiment, various modifications are possible. For example, the adhesive material may be applied to only one side (the lower side in FIG. 2) of the attachment part 160 that may be in contact with the body of the user, and the other side (the upper side in FIG. 2) of the attachment part 160 corresponding thereto may be inserted into and connected to the base part 110.

Referring to FIG. 2, the alarm unit 170 according to an embodiment of the present disclosure is electrically connected to the substrate part 112 and may receive an electrical signal from the substrate part 112 and generate sound.

There is an effect in which the substrate part 112 may receive an electrical signal from the sensor part (not shown) and may transmit an electrical signal to the alarm unit 170 according to the operating state of the liquid medicine injection device 1 when necessary, for example, in an emergency situation, and as the sound is generated from the alarm unit 170, the user may recognize a specific state.

Referring to FIG. 1, the lever part 190 according to an embodiment of the present disclosure is arranged on the cover part 150 and may be rotatably connected to the cover part 150. The lever part 190 may be connected to the needle part 130. As the user rotates the lever part 190, the needle part 130 connected to the lever part 190 may rotate in conjunction with the lever part 190, and the needle body provided in the needle part 130 may penetrate into the skin of the user.

The operation principle and effects of the liquid medicine injection device 1 according to an embodiment of the present disclosure are described.

Referring to FIGS. 1 to 10, the liquid medicine injection device 1 according to an embodiment of the present disclosure may include the base part 110, the reservoir part 120, the needle part 130, the driving part 140, the cover part 150, the attachment part 160, the alarm unit 170, the battery part 180, and the lever part 190.

The user may remove the cover attached to the other surface (the lower surface in FIG. 1) opposite to one surface of the attachment part 160 connected to the base part 110, and may attach the attachment part 160 to the skin of the user in a state where an adhesive material is applied thereto.

After attaching the liquid medicine injection device 1 to the skin of the user, the user rotates the lever part 190 such that the needle body provided in the needle part 130 penetrates into the skin of the user. Although not illustrated in the drawings, a sensor needle (not shown) provided in the sensor part may penetrate into the skin of the user and measure a blood glucose level in the body of the user.

Power may be supplied from the battery part 180 installed in the base part 110, and an electrical signal may be transmitted to the driving part 140 through the substrate part 112.

As the electrical signal is applied to the driving part 140, an electrochemical reaction occurs in the fluid contained in the driving housing 141, and power may be generated by pressure that is generated as the fluid flows between the first space and the second space respectively formed on both sides of the membrane arranged inside the driving housing 141.

Due to this, the shaft part 145 may reciprocate while moving forward and backward along the longitudinal central axis AX within the protrusion part 142 formed in the driving housing 141.

Referring to FIGS. 3 to 7, the protrusion part 142 and the reinforcement part 143 formed to protrude outward from the protrusion part 142 and having a certain thickness may be arranged outside the first part 145a1 that is connected to the shaft head 145b and passes through the shaft hole 141H in the shaft body 145a according to an embodiment of the present disclosure.

Referring to FIG. 7, the thickness of a certain portion of the protrusion part 142 where the reinforcement part 143 is formed may be formed to be relatively greater than the diameter of the shaft body 145a. This provides an effect of preventing one end portion of the shaft body 145a from rotating in a clockwise or counterclockwise direction with a certain point on the longitudinal central axis AX as the center of rotation, and improving straightness of the shaft part 145 that reciprocates along the longitudinal central axis AX.

In addition, referring to FIGS. 8 and 9, the rib part 114 may be formed to have a certain thickness on the base part 110, specifically the second body 113, where the driving part 140 is arranged, and the guide part 115 may be formed in the rib part 114 in the shape of a groove part.

Referring to FIGS. 8 to 10, the guide part 115 may be open on one side (in FIG. 10) and may be formed in the shape of a groove part. The second part 145a2 formed on the side opposite to the shaft body 145a, specifically the first part 145a1 to which the shaft head 145b is connected, is arranged inside the guide part 115. Accordingly, it is possible to prevent the longitudinal central axis AX of the shaft body 145a from being distorted.

Specifically, since the outer circumferential surface of the shaft body 145a and the inner circumferential surface of the guide part 115 are spaced apart from each other by the separation distance SD at a plurality of points in the circumferential direction with respect to the center of the shaft body 145a, there is an effect of preventing the longitudinal central axis AX of the shaft body 145a from being distorted beyond the separation distance SD, and there is an effect of improving the reciprocating motion straightness of the shaft part 145.

In addition, since the outer circumferential surface of the shaft body 145a and the inner circumferential surface of the guide part 115 are spaced apart from each other by the separation distance SD, it is possible to prevent the driving force of the driving part 140 from being reduced due to the contact and friction between the shaft body 145a and the guide part 115.

In the liquid medicine injection device 1 according to an embodiment of the present disclosure, since the shaft part 145 reciprocates within the guide part 115 formed in the shape of a groove part in the rib part 114 provided in the base part 110, specifically the second body 113, there is an effect of improving straightness and improving precision in transmitting power from the driving part 140 to the reservoir part 120.

In addition, since it is possible to prevent the longitudinal central axis of the shaft part 145 from being distorted, there is an effect of maintaining airtightness between the outer circumferential surface of the shaft head 145b and the inner circumferential surface of the protrusion part 142 formed in the driving housing 141, and preventing the fluid contained in the driving housing 141 from leaking out through the shaft hole 141H.

It will be understood that the concept of the present disclosure should not be limited to the embodiments described above, and the claims and all equivalent modifications fall within the scope of the present disclosure.

### Industrial Applicability

According to the present disclosure, a liquid medicine injection device is provided. In addition, embodiments of the present disclosure may be applied to industrially applicable devices that inject liquid medicine into the body of a patient.

## Claims

1. A liquid medicine injection device comprising:
a needle part which is provided with one or more needle bodies formed to be injected into a body of a user;
a reservoir part in which liquid medicine to be delivered to the needle part is contained;
a driving part which is provided with a shaft part capable of performing a reciprocating motion in a preset direction and transmits power to the reservoir part such that the liquid medicine contained in the reservoir part is delivered to the needle part; and
a base part in which the reservoir part, the needle part, and the driving part are installed,
wherein the base part is provided with a rib part in which a guide part is formed in a shape of a groove part, and the shaft part is capable of performing the reciprocating motion in the guide part.

2. The liquid medicine injection device of claim 1, wherein the base part comprises:
a first body arranged on one side of each of the reservoir part, the needle part, and the driving part; and
a second body arranged on the first body and fixing positions of the reservoir part, the needle part, and the driving part.

3. The liquid medicine injection device of claim 2, wherein the rib part is formed to have a certain thickness on the second body.

4. The liquid medicine injection device of claim 1, wherein the driving part comprises:
a driving housing having a shaft hole formed therein;
the shaft part passing through the shaft hole and extending to an outside of the driving housing; and
a moving part connected to the shaft part and movable in conjunction with the reciprocating motion of the shaft part.

5. The liquid medicine injection device of claim 4, wherein the driving part further comprises a stopper part connected to the shaft part and arranged on either side of the moving part.

6. The liquid medicine injection device of claim 4, wherein a protrusion part is formed to extend on one side of the driving housing while sharing a longitudinal central axis with the shaft part and is provided with the shaft hole.

7. The liquid medicine injection device of claim 6, wherein a reinforcement part is formed to have a certain thickness and protrude outward on one surface of the protrusion part where the shaft hole is formed.

8. The liquid medicine injection device of claim 1, further comprising a cover part that covers the base part.

9. The liquid medicine injection device of claim 8, further comprising a lever part rotatably installed in the cover part and transmitting rotational power to the needle part.

10. The liquid medicine injection device of claim 1, further comprising an attachment part connected to the base part and attachable to the user.
